# EUROPEAN PATENT SPECIFICATION

(11) **EP 3 323 413 B1**
(45) Date of publication and mention of the grant of the patent: **02.09.2020**
(21) Application number: 16824623.9
(22) Date of filing: 29.06.2016
(51) Int. Cl.: A61K 31/415, A61K 31/135, A61K 47/38, A61K 47/44, A61K 47/36, A61K 9/24

(54) **PHARMACEUTICAL COMPOSITION CONTAINING CELECOXIB AND TRAMADOL**
PHARMAZEUTISCHE ZUSAMMENSETZUNG MIT CELECOXIB UND TRAMADOL
COMPOSITION PHARMACEUTIQUE CONTENANT DU CÉLÉCOXIB ET DU TRAMADOL

(30) Priority: 14.07.2015 KR 20150099808
(43) Date of publication of application: 23.05.2018
(73) Proprietor: Yoo Young Pharm Co., Ltd., Seoul 06687 (KR)
(72) Inventor: KIM, Jung Ju, Seoul 06093 (KR); LEE, Dong Min, Hwaseong-si Gyeonggi-do 18477 (KR); KIM, Sun Kyoung, Incheon 21125 (KR)
(74) Representative: Louis Pöhlau Lohrentz
(86) International application number: PCT/KR2016/006930
(87) International publication number: WO 2017/010706

(56) References cited:
- WO-A1-2009/049405
- WO-A1-2015/016695
- JP-A- 2014 221 797
- KR-A- 20040 067 969
- KR-A- 20080 104 910
- KR-A- 20110 122 287
- KR-A- 20130 069 484
- US-A1- 2009 175 939

## Description

### [Technical Field]

The present invention relates to a pharmaceutical composition containing celecoxib known as a selective COX-2 inhibitor and tramadol which is an opioid analgesic.

### [Background Art]

### Pain

According to the definition of the International Association for the Study of Pain, pain is defined as an unpleasant sensory and emotional experience associated with actual or potential tissue damage. The causes of pain can largely be divided into two: a perceptual case caused by damage or inflammation of somatic or visceral tissue; and a neuropathic case resulting from nerve injury. Among these, an onset mechanism reported regarding the perceptual case is as follows.

If tissue is damaged, the damage stimulus itself will excite A-delta and C-nociceptors to transmit pain. Subsequently, bradykinin, serotonin, K⁺, H⁺, and substance P are released from the damaged tissue cells and peripheral nerve endings. In this process, sensitization may occur in the peripheral nociceptors of the injured site, resulting in lower threshold and multiple excitements. This pain signal may be transmitted to the spinal cord through the pain sensory nerve, and be raised to the upper part of the spinal cord and processed, thereby causing pain. In addition, if tissue is damaged, the cell membrane is destroyed and phospholipase A is activated to produce arachidonic acid. Then, prostaglandin is synthesized from the arachidonic acid through the cyclooxygenase (COX) pathway. The prostaglandin may also sensitize peripheral nociceptors to cause pain (Study on Pain Mechanism, BioWave Vol. 8 No. 3 2006).

### Pain-Related Drugs

For reducing pain, analgesics are used to lower the intensity of pain, and are sometimes used in combination with antidepressants because chronic pain can cause depression. Among these, representative analgesics include opioid analgesics, which are narcotic analgesics, and non-steroidal anti-inflammatory drugs (NSAIDs) which are non-narcotic analgesics.

Opioid analgesics are opioid compounds and are known to bind to opioid receptors to block the secretion of neurotransmitters such as P-substance and glutamate so as to prevent the pain signal from being transmitted to the brain, thereby strongly inhibiting pain. However, in recent years, it has been reported that long-term use of opioid drugs causes side effects such as drug poisoning, bone fracture risk, myocardial infarction, and sexual dysfunction. Thus, the need to reduce the dose of opioid drugs has been addressed.

Non-steroidal anti-inflammatory drugs are known to inhibit cyclooxygenase so as to inhibit the synthesis of prostaglandins, thereby inhibiting inflammation and pain. Initially, non-steroidal anti-inflammatory drugs such as ibuprofen, diclofenac or naproxen have been used. However, since it was reported that these drugs may inhibit even COX-1 to cause gastrointestinal diseases, the COX-2 selective inhibitor, such as celecoxib has attracted attention. However, these drugs indirectly inhibit pain through their anti-inflammatory action, and thus the analgesic effects thereof are not as strong as those of the above-described opioid analgesics.

### Co-Administration of Celecoxib and Tramadol

As an alternative to increase the analgesic effect of a COX-2 selective inhibitor, Korean Patent No. 10-0444195 paid attention on the synergistic effect of co-administration of a COX-2 selective inhibitor and an opioid analgesic. According to the above patent document, the analysis of an equieffective dose substitution model and a curvilinear regression utilizing all the data for COX-2 selective inhibitor and opioid analgesic establishes the existence of unexpectedly enhanced analgesic activity of combinations of COX-2 inhibitor and opioid analgesic. Therefore, the co-administration of the two drugs can reduce the dose compared with the dose when the two medicines were used alone for the same pain, thereby reducing the number of kind and degree of side effects caused by each drug.

Korean unexamined Patent Application Publication No. 10-2012-0089287 paid attention on the synergistic effect of tramadol and celecoxib in order to avoid side effects resulting from high doses and repeated use of opioid analgesics. The above document discloses that a combination of tramadol and celecoxib is effective for the treatment of severe pain or moderate pain, particularly pain with inflammatory factors, and is particularly effective against diseases, disorders or related pain, such as sciatica, frozen shoulder or central sensitization (e.g., central pain syndrome), against which the effects of simple single drugs are insufficient.

### Characteristics in Formulation of Each of Celecoxib and Tramadol

Celecoxib is a compound having the chemical name 4-[5-(4-methylphenyl)-3-(trifluoromethyl)-1H-pyrazol-1-yl]benzenesulfonamide and the following formula 1:

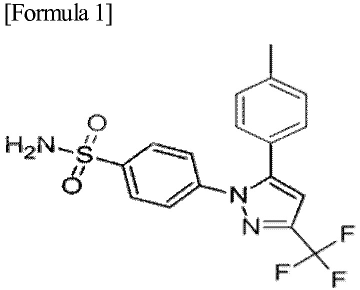

Celecoxib is a poorly soluble drug which is not easy to formulate. In order to increase the bioavailability of celecoxib, Korean Patent No. 10-0501034 attempted to atomize celecoxib particles, and Korean Patent No. 10-1455901 used poloxamer as a surfactant (so called "solubilizer"), and Korean Patent No. 10-1237646 used a solid dispersion technique that modifies the surface of celecoxib particles with a water-soluble polymer and a surfactant.

Tramadol is a compound having the chemical name 2-[(dimethylamino)methyl]-1-(3-methoxyphenyl)cyclohexanol and the following formula 2:

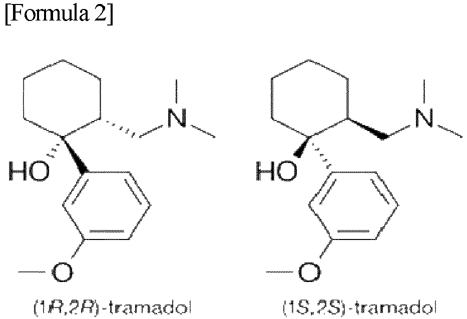

Since tramadol is a water-soluble drug that is mainly used for chronic pain, it is effective to formulate tramadol in a sustained-release form in order to improve convenience of the drug and the like. In order to formulate tramadol in a sustained-release form, in Korean Patent No. 10-1455741, a gel-forming substance capable of controlling the release of an active ingredient by forming a hydrogel upon contact with water was added, such as a cellulose derivative or a carboxyvinyl polymer, for example, hydroxypropyl cellulose, methylcellulose, hydroxypropyl methylcellulose, sodium carboxymethylcellulose or the like.

US 2009/0175939 A1 discloses pharmaceutical compositions.

WO 2009/049405 A1 discloses a bilayer composition for the sustained release of acetaminophen and tramadol.

WO 2015/016695 A1 discloses a novel biphasic-delivery pharmaceutical system for the treatment of pain and inflammation.

### [Disclosure]

### [Technical Problem]

The present inventors have paid attention on the synergistic effect of a combination of celecoxib and tramadol against pain, and have attempted to formulate the two different active ingredients in a single dosage form in order to increase compliance of each drug.

For this purpose, the first consideration was the design of release pattern of each drug. Numerous repetitive experiments have shown that it is preferable that celecoxib be present as an immediate-release compartment and tramadol be present as a sustained-release compartment.

Next, the present inventors concerned about the type of formulation that can achieve the above-described release patterns, but it was thought that various types of formations could be applicable. Examples of applicable formulations include: a cored tablet comprising an inner core consisting of a sustained-release compartment, and an outer layer consisting of an immediate-release compartment; a capsule comprising a particle, granule, pellet or tablet consisting of a sustained-release compartment, and a particle, granule, pellet or tablet consisting of an immediate-release compartment; and a multilayer tablet comprising a layer consisting of a sustained-release compartment, and a layer consisting of an immediate-release compartment.

Among these formulations, the multilayer tablet was selected as a preferred example, and then a sustained-release matrix of tramadol and an immediate-release matrix of celecoxib were stacked, and then, the release pattern of each active ingredient was tested. As a result, there was a problem in achieving the desired dissolution of celecoxib.

Accordingly, the present inventors have conducted many repetitive experiments to develop a novel formulation capable of achieving the desired release patterns of celecoxib and tramadol even in a single dosage form, and as a result, have completed the present invention.

### [Technical Solution]

The present invention solves the above-described problem a pharmaceutical composition as claimed in claim 1.

Preferred embodiments are recited in claims 2 to 6.

(11) The object of the invention is also solved by the pharmaceutical composition of any one of claims 1 to 6, wherein the first compartment and the second compartment are composed of separate layers which are stacked on each other.

### [Advantageous Effects]

The present invention is directed to an oral solid combination formulation showing drug release patterns similar to those shown when a commercially available oral solid single formulation of celecoxib and a commercially available oral solid single formulation of tramadol are respectively administered at the doses which are same with those of the combination formulation of the present invention. Specifically, the present invention relates to a formulation obtained by formulating celecoxib and tramadol in a single dosage form, wherein celecoxib that is a poorly soluble drug is solubilized so as to be released in an immediate manner, and tramadol is configured to be released in a sustained manner. In addition, the interaction between the drugs in the formulation is minimized. Thus, the formulation of the present invention is a combination formulation designed such that the effects of the drugs are complementary to each other and long-lasting even when the formulation is administered once a day.

In addition, a multilayer table embodied according to the present invention has excellent hardness and interlayer adhesion, and thus is easy to package, transport and handle. Furthermore, it is suitable for mass production because tablet defect, such as capping or laminating less occur.

### [Description of Drawings]

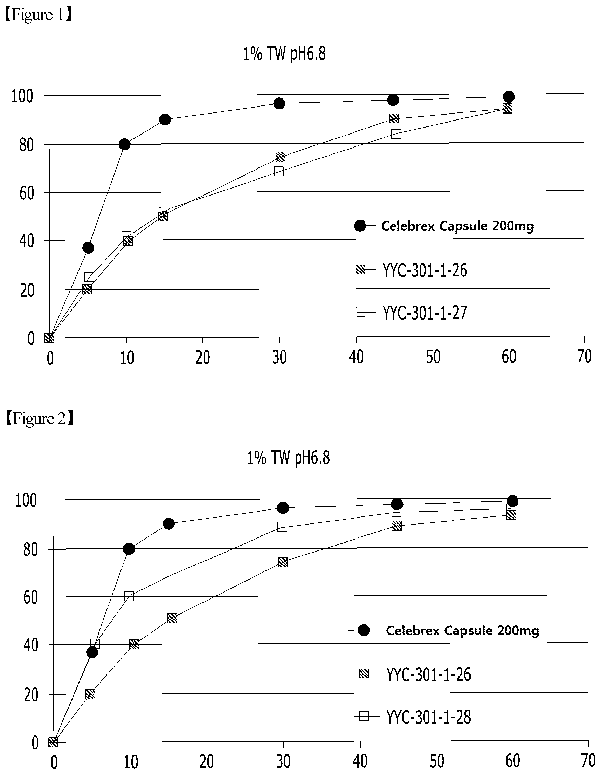

FIGS. 1 to 13 show the dissolution pattern of each example.

### [Mode for Invention]

The present invention is directed to an oral solid combination formulation containing: a celecoxib compartment in an immediate-release composition; and a tramadol compartment in a sustained-release composition. When two or more drugs that need to exhibit different release patterns are formulated in a single dosage form, it is important to design the formulation such that the composition of any compartment does not affect the release pattern of the drug of the other compartment. In particular, when celecoxib is to be formulated in a single dosage form, it is necessary to add special solubilizing means because the drug itself is very poorly soluble. Furthermore, it is a very sensitive drug having difficulty in achieving satisfactory dissolution.

For this reason, when a known sustained-release tramadol compartment and a celecoxib compartment were simply brought into contact with each other, the sustained-release composition of the tramadol compartment adversely affected the release pattern of celecoxib as expected. Perhaps for this reason, the release pattern of celecoxib was unsatisfactory, even though a solubilizing means was provided within the celecoxib compartment.

Accordingly, the present inventors have focused on the composition of a sustained-release tramadol compartment and the composition of a celecoxib compartment so as to minimize the influence of the composition of the tramadol compartment on the release pattern of celecoxib.

### Definition

"Celecoxib" and "tramadol" refer to possible forms that can exhibit the well-known pharmacological activities of celecoxib and tramadol during drug metabolism after administration. Non-limiting examples include a free acid/base, salt, co-crystal, racemate and prodrug of each of celecoxib and tramadol. For example, "tramadol" can also be interpreted as tramadol hydrochloride.

"Solubilizing means" refers to one of known methods for improving the dissolution of poorly soluble drugs. Non-limiting examples of known solubilizing means for celecoxib include particle atomization, poloxamer addition, and solid dispersion.

"Water-soluble polymer" refers to a resin or polymer substance which can be dissolved or swelled or dispersed into small particles in water. Non-limiting examples of the water-soluble polymer include hydroxypropylmethylcellulose (HPMC), hydroxypropylcellulose (HPC), and polyvinylpyrrolidone (PVP).

"Surfactant" refers to a substance that has both a hydrophilic group and a lipophilic group in the molecule, and can be dissolved or dispersed in a solvent and selectively adsorbed to an interface, thereby significantly changing the properties of the interface. Non-limiting examples of the surfactant include sodium lauryl sulfate (SLS) and poloxamer.

"Saccharide" refers to a carbohydrate compound which has a relatively small molecular weight and dissolves in water to give a sweet taste. Non-limiting examples of the saccharide include mannitol and maltitol.

"Water-insoluble polymer" refers to a polymer substance that does not dissolve or swell in water. Non-limiting examples of the water-insoluble polymer include ethyl cellulose and cellulose acetate.

"Wax-like lipid" refers to a lipid having properties similar to those of wax. Non-limiting examples of the wax-like lipid include glyceryl fatty acid ester and fatty acid macrogol glyceride.

Unless otherwise specified, the specific components of the various additives mentioned herein, including "water-soluble polymer", "surfactant", "saccharide", "water-insoluble polymer" and "wax-like lipid", can be suitably selected from among pharmaceutically acceptable substances known in the HANDBOOK OF PHARMACEUTICAL EXCIPIENTS, etc.

### First Compartment Comprising Celecoxib

The first compartment comprises one or more selected from among a water-soluble polymer, a surfactant and a saccharide.

The water-soluble polymer is one or more selected from among hydroxypropyl cellulose, hydroxypropyl methylcellulose, and a vinylpyrrolidone-vinyl acetate copolymer. Most preferably, the water-soluble polymer is hydroxypropyl cellulose. The surfactant preferably comprises one or more selected from among polyoxyglyceride, polyoxyethylene sorbitan fatty acid ester, sodium lauryl sulfate, glyceryl fatty acid ester, fatty acid macrogol glyceride, diethylene glycol monoethyl ether, and sucrose fatty acid ester. The saccharide comprises one or more selected from among mannitol, maltitol, lactitol, ribitol, inositol, xylitol, maltotritol, and glucose.

Each of the water-soluble polymer and the surfactant is preferably contained in an amount of 0.1 to 20 wt% of the total weight of the first compartment. If the content of each of the water-soluble polymer and the surfactant is out of the above-described range, the dissolution rate of celecoxib can be reduced by about 10% or more. Above all, if the content of the surfactant does not satisfy the lower limit and upper limit of the above-described range, the surfactant activity can be significantly reduced by about 10% or more.

The saccharide is preferably contained in an amount of 10 to 50 wt% of the total weight of the first compartment. If the content of the saccharide does not satisfy the lower limit and the upper limit of the above-described range, sufficient solubilization of celecoxib cannot be achieved, and a problem may arise in the tableting process or tablet size increases.

The first compartment may also comprise suitable amounts of other known additives that can be suitably selected within a range that does not impair the desired effect of the present invention.

### Second Compartment Comprising Tramadol

Generally, means for delaying drug release include a method of coating the drug with a water-insoluble substance or a method of dispersing the drug in a matrix material which is water-insoluble and water-permeable. Examples of the water-insoluble substance include white wax, carnauba wax, shellac, cellulose derivatives, glyceryl monostearate, glyceryl tristearate, synthetic hydrogel, and the like.

In addition, as disclosed in Korean Patent No. 10-1455741, drug release can also be delayed by using a gel-forming substance such as a cellulose derivative or a carboxyvinyl polymer, for example, hydroxypropyl cellulose, methylcellulose, hydroxypropyl methylcellulose, sodium carboxymethylcellulose or the like, which is capable of controlling the release of an active ingredient by forming a hydrogel upon contact with water

However, the present inventors have found that the known substances as described above, when brought into contact with the first compartment in a single formulation, can induce interaction with the first compartment to adversely affect the dissolution of celecoxib, even though these substances may be preferable in terms of releasing tramadol itself in a sustained manner.

Accordingly, the present inventors have made extensive efforts to find a novel formulation, and as a result, have surprisingly found that specific water-insoluble substances have less influence on the release pattern of celecoxib of the first compartment. In addition, the present inventors have found that combining a particular water-insoluble substance with a wax-like lipid rather than simply selecting only one kind of water-insoluble substance is most preferable in terms of achieving the desired release pattern of each drug of the first and second compartments.

The second compartment comprises a water-insoluble polymer and a wax-like lipid.

The water-insoluble polymer is one or more selected from cellulose-based polymers. More preferably, the water-insoluble polymer is ethyl cellulose.

The wax-like lipid is one or more selected from among glycerol stearate, glycerol behenate, glycerol palmitostearate, fatty acid macrogol glyceride, diethylene glycol monoethyl ether, glyceryl monocaprylate, and hydrogenated castor oil.

Each of the wax-like lipid and the water-insoluble polymer is preferably contained in an amount of 1 to 60 wt% of the total weight of the second compartment. If the content of each of the wax-like lipid and the water-insoluble polymer does not satisfy the upper and lower limits of the above-described range, there is a possibility that drug release is hardly achieved within 12-24 hours.

The second compartment may also comprise suitable amounts of other known additives that can be suitably selected within a range that does not impair the desired effect of the present invention.

### Formulation

Non-limiting examples of a final formulation include: a cored tablet comprising an inner core consisting of a sustained-release compartment, and an outer layer consisting of an immediate-release compartment; a capsule comprising a particle, granule, pellet or tablet consisting of a sustained-release compartment, and a particle, granule, pellet or tablet consisting of an immediate-release compartment; and a multilayer tablet comprising a layer consisting of a sustained-release compartment, and a layer consisting of an immediate-release compartment.

### Dosage · Administration

Celecoxib is generally taken 200 mg once a day. Thus, commercially available single formulations include products containing 100 mg of celecoxib, and products containing 200 mg of celecoxib. Tramadol may be administered at various doses, but is generally taken 100-200 mg daily. Thus, commercially available formulations include products containing 50 mg of tramadol, and products containing 100 mg of tramadol.

The combination formulation according to the present invention can be designed to have various doses and can be taken in an appropriate manner. However, according to one embodiment of the present invention, celecoxib is provided as a solubilized immediate-release layer in order to exhibit an initial pain relief effect, and tramadol is provided as a sustained-release layer in order to reduce side effects and exhibit a long-lasting pain relief effect. To this end, the contents of the drugs in one formulation are set at 200 mg celecoxib and 100 mg tramadol, and this combination formulation can exhibit a satisfactory pain relief effect even when it is administered once a day.

As described in the above-described embodiment of the present invention, when the content of tramadol in the combination formulation for once-daily administration is designed to be 100 mg, a sufficient pain relief effect is obtained due to the synergistic effect of tramadol with celecoxib, even though the amount of tramadol is small. Particularly, in this case, there is an advantage that side effects associated with tramadol can be reduced.

However, the above described dosage regimen is merely a preferred embodiment, and the scope of the present invention is not limited thereto.

Hereinafter, the present invention will be described with reference to examples. It is to be understood, however, that these examples are not intended to limit the scope of the present invention in any way.

### Fundamental Conditions

Celecoxib used in the examples of the present invention has a D90 of 10 to 15 µm and a D50 of 4 to 7 µm.

"200 mg Celebrex ™ capsule" and "100 mg Tridol ™ sustained-release tablet" refer to commercially available single formulations of celecoxib and tramadol, respectively.

The approximate main components of the product names used in the examples are shown in Table 1 below.

**Table 1**

| Product name | Component name |
|---|---|
| Ryoto sugar ester P-1570 | **Sucrose palmitate** |
| Ryoto sugar ester S-1570 | **Sucrose stearate** |
| Ryoto sugar ester L-1695 | Sucrose laurate |
| Ryoto sugar ester L-10D | Sucrose laurate |
| Labrafil M2125CS | Linoleoyl macrogol-6-glyceride |
| Labrafil M1994CS | Oleoyl macrogol-6-glyceride |
| Peceol | Glycerol monooleates |
| Transutol P | Diethylene glycol monoethyl ether |
| Celucire 44/14 | Lauroyl macrogol-32-glyceride |
| Capmul MCM(C8) | Glyceryl monocaprate |
| SLS | Sodium lauryl sulfate |
| Pharmacoat 606 | Hydroxypropylmethylcellulose |
| PVP-K30 | Polyvinylpyrrolidone |
| PVP-K25 | Polyvinylpyrrolidone |
| Kollidon VA64 | Vinylpyrrolidone-vinyl acetate copolymer |
| Natrosol 250H(HEC) | Hydroxyethylcellulose |
| Pharmacoat 603 | Hydroxypropylmethylcellulose |
| Pharmacoat 645 | Hydroxypropylmethylcellulose |
| PEG6000 | Polyethyleneglycol 6000 |
| Soluplus | Polyvinyl caprolactam-polyvinyl acetate-polyethylene glycol graft copolymer |
| HPC-L | Hydroxypropylcellulose |
| Pearitol 100SD | D-mannitol |
| SMCC90 | Silicified microcrystallin cellulose |
| Primellose | Crosscarmellose sodium |
| Mg-St | Magnesium stearate |
| Castor oil | Castor oil |
| HP-β-CD | 2-hydroxypropyl-β-cyclodextrin |
| Emdex | Deatrates |
| Aqualon EC N7 | Ethylcellulose |
| Aqualon EC N14 | Ethylcellulose |
| Compritol 888ATO | Glycerol dibehenate |
| Precirol ATO5 | Glycerol distearate |
| SMCC50 | Silicified microcrystallin cellulose |
| Syloid | Silicon dioxide |
| Lubritab | Hydrogenated vegetable oil |
| PH101 | Microcrystalline cellulose |
| Starch1500 | Pregelatinized starch |
| EC(100cP) | Ethylcellulose |
| EC(7cP) | Ethylcellulose |
| HPMC2910 15000SR | Hydroxypropylmethylcellulose |
| Kollidon CL | Crospovidone |

### Example 1: Formulation after Solublization of Poorly Soluble Drug

### Example 1-1: Formulation Using Water-Soluble Polymer, Surfactant and Saccharide - Evaluation of Dissolution Pattern according to the Proportions of Water-Soluble Polymer and Surfactant

Celecoxib and the excipients shown in Table 2 below were wet-kneaded, dried, granulated, mixed, and compressed into a tablet by wet-granulation method, and the tablet was subjected to an *in vitro* dissolution test.

**Table 2**

| Process | Function | Component name | YYC-301-1-26 | | YYC-301-1-27 | |
|---|---|---|---|---|---|---|
| | | | mg | % | mg | % |
| Kneading | Active | Celecoxib | 200 | 57.14 | 200 | 57.14 |
| | ingredient | | | | | |
| | Excipient | mannitol100SD | 50 | 14.29 | 50 | 14.29 |
| | Excipient | Pharmacoat 603 | 5 | 1.43 | 10 | 2.86 |
| | Excipient | Sugar ester P-1570 | 5 | 1.43 | 10 | 2.86 |
| | Excipient | SMCC90 | 79.5 | 22.71 | 69.5 | 19.86 |
| | Disintegrant | Primellose | 7 | 2 | 7 | 2 |
| | Disintegrant | Mg-St | 3.5 | 1 | 3.5 | 1 |
| Binding solvent | | Water | 78 | | 116 | |
| Sum | | | 350 | 100 | 350 | 100.01 |

Only the celecoxib granules in Table 2 above were filled in a capsule, and dissolution of the capsule was evaluated. The results are shown in Table 3 below and FIG. 1.

**Table 3**

| Time (min) | Celebrex capsule 200 mg | | YYC-301-1-26 | | YYC-301-1-27 | |
|---|---|---|---|---|---|---|
| | Dissolution rate | SD | Dissolution rate | SD | Dissolution rate | SD |
| 0 | 0.00 | 0.00 | 0.00 | 0.00 | 0.00 | 0.00 |
| 5 | 37.13 | 8.01 | 20.86 | 0.03 | 24.14 | 1.18 |
| 10 | 79.99 | 6.64 | 38.66 | 2.69 | 41.46 | 1.02 |
| 15 | 89.75 | 5.75 | 50.74 | 0.35 | 51.96 | 0.82 |
| 30 | 96.42 | 2.77 | 74.36 | 0.11 | 68.93 | 0.87 |
| 45 | 97.48 | 2.43 | 89.49 | 0.55 | 83.40 | 1.94 |
| 60 | 98.02 | 2.23 | 93.78 | 0.52 | 93.32 | 0.23 |

### Example 1-2: Formulation Using Water-Soluble Polymer, Surfactant and Saccharide - Evaluation of Dissolution Pattern according to the Amount of Saccharide

Celecoxib and the excipients shown in Table 4 below were wet-kneaded, dried, granulated, mixed, and compressed into a tablet by wet-granulation method, and the tablet was subjected to an *in vitro* dissolution test.

**Table 4**

| Process | Function | Component name | YYC-301-1-26 | | YYC-301-1-28 | |
|---|---|---|---|---|---|---|
| | | | mg | % | mg | % |
| Kneading | Active ingredient | Celecoxib | 200 | 57.14 | 200 | 57.14 |
| | Excipient | mannitol100SD | 50 | 14.29 | 80 | 22.86 |
| | Excipient | Pharmacoat 603 | 5 | 1.43 | 5 | 1.43 |
| | Excipient | Sugar ester P-1570 | 5 | 1.43 | 5 | 1.43 |
| | Excipient | SMCC90 | 79.5 | 22.71 | 49.5 | 14.14 |
| | Disintegrant | Primellose | 7 | 2 | 7 | 2 |
| | Disintegrant | Mg-St | 3.5 | 1 | 3.5 | 1 |
| Binding solvent | | Water | 78 | | 78 | |
| Sum | | | 350 | 100 | 350 | 100 |

Only the celecoxib granules in Table 4 above were filled in a capsule, and dissolution of the capsule was evaluated. The results are shown in Table 5 below and FIG. 2.

**Table 5**

| Time (min) | Celebrex capsule 200 mg | | YYC-301-1-26 | | YYC-301-1-28 | |
|---|---|---|---|---|---|---|
| | Dissolution rate | SD | Dissolution rate | SD | Dissolution rate | SD |
| 0 | 0.00 | 0.00 | 0.00 | 0.00 | 0.00 | 0.00 |
| 5 | 37.13 | 8.01 | 20.86 | 0.03 | 39.58 | 1.18 |
| 10 | 79.99 | 6.64 | 38.66 | 2.69 | 60.85 | 1.02 |
| 15 | 89.75 | 5.75 | 50.74 | 0.35 | 68.67 | 0.82 |
| 30 | 96.42 | 2.77 | 74.36 | 0.11 | 88.94 | 0.87 |
| 45 | 97.48 | 2.43 | 89.49 | 0.55 | 95.03 | 1.94 |
| 60 | 98.02 | 2.23 | 93.78 | 0.52 | 96.87 | 0.23 |

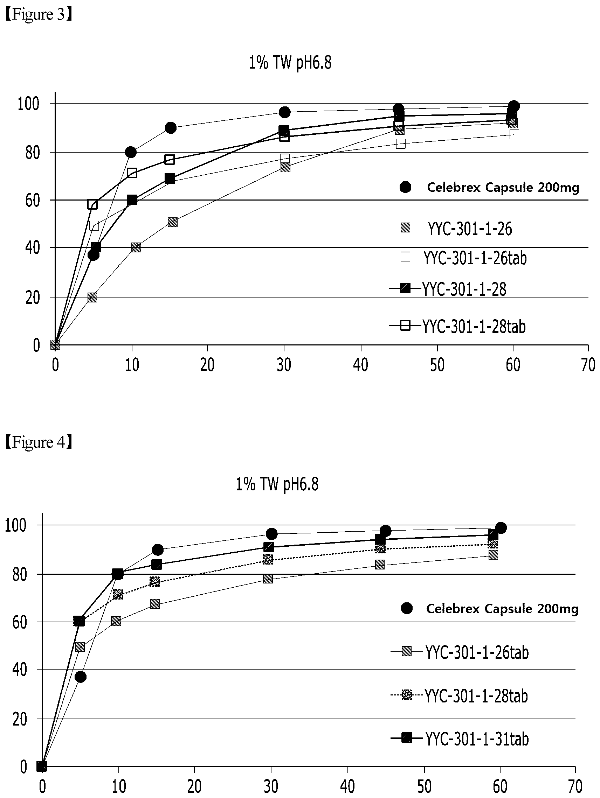

The celecoxib granules in Table 4 above were subjected to post-mixing and compressed into a tablet, and dissolution of the tablet was evaluated. The results are shown in Table 6 below and FIG. 3.

**Table 6**

| Time (min) | Celebrex capsule 200 mg | | YYC-301-1-26 | | YYC-301-1-26tab | | YYC-301-1-28tab | | YYC-301-1-28tab | |
|---|---|---|---|---|---|---|---|---|---|---|
| | Dissolution rate | SD | Dissolution rate | SD | Dissolution rate | SD | Dissolution rate | SD | Dissolution rate | SD |
| 0 | 0.00 | 0.00 | 0.00 | 0.00 | 0.00 | 0.00 | 0.00 | 0.00 | 0.00 | 0.00 |
| 5 | 37.13 | 8.01 | 20.86 | 0.03 | 49.26 | 1.36 | 39.58 | 2.02 | 59.10 | 3.04 |
| 10 | 79.99 | 6.64 | 38.66 | 2.69 | 60.70 | 0.77 | 60.85 | 2.87 | 71.02 | 1.49 |
| 15 | 89.75 | 5.75 | 50.74 | 0.35 | 67.61 | 0.47 | 68.67 | 1.08 | 76.92 | 0.82 |
| 30 | 96.42 | 2.77 | 74.36 | 0.11 | 77.70 | 0.29 | 88.94 | 2.96 | 85.93 | 0.10 |
| 45 | 97.48 | 2.43 | 89.49 | 0.55 | 83.73 | 0.06 | 95.03 | 1.92 | 90.86 | 0.28 |
| 60 | 98.02 | 2.23 | 93.78 | 0.52 | 87.53 | 0.07 | 96.87 | 0.82 | 93.13 | 0.36 |

Celecoxib and the excipients shown in Table 7 below were wet-kneaded, dried, granulated, mixed, and compressed into a tablet by wet-granulation method, and the tablet was subjected to an *in vitro* dissolution test.

**Table 7**

| Process | Function | Component Name | YYC-301-1-26 | | YYC-301-1-28 | | YYC-301-1-31 | |
|---|---|---|---|---|---|---|---|---|
| | | | mg | % | mg | % | mg | % |
| Kneading | Active ingredient | Celecoxib | 200 | 57.14 | 200 | 57.14 | 200 | 58.82 |
| | Excipient | mannitol100SD | 50 | 14.29 | 80 | 22.86 | 119.8 | 35.24 |
| | Excipient | Pharmacoat 603 | 5 | 1.43 | 5 | 1.43 | 5 | 1.47 |
| | Excipient | Sugar ester P-1570 | 5 | 1.43 | 5 | 1.43 | 5 | 1.47 |
| | Excipient | SMCC90 | 79.5 | 22.71 | 49.5 | 14.14 | | |
| | Disintegrant | Primellose | 7 | 2 | 7 | 2 | 6.8 | 2.0 |
| | Disintegrant | Mg-St | 3.5 | 1 | 3.5 | 1 | 3.4 | 1.0 |
| Binding solvent | | Water | 78 | | 78 | | 92.5 | |
| Sum | | | 350 | 100 | 350 | 100 | 340 | |

The celecoxib granules in Table 7 above were subjected to post-mixing and compressed into a tablet, and dissolution of the tablet was evaluated. The results are shown in Table 8 below and FIG. 4.

**Table 8**

| Time (min) | Celebrex capsule 200 mg | | YYC-301-1-26tab | | YYC-301-1-28tab | | YYC-301-1-31tab | |
|---|---|---|---|---|---|---|---|---|
| | Dissolution rate | SD | Dissolution rate | SD | Dissolution rate | SD | Dissolution rate | SD |
| 0 | 0.00 | 0.00 | 0.00 | 0.00 | 0.00 | 0.00 | 0.00 | 0.00 |
| 5 | 37.13 | 8.01 | 49.26 | 1.36 | 59.10 | 3.04 | 60.23 | 6.41 |
| 10 | 79.99 | 6.64 | 60.70 | 0.77 | 71.02 | 1.49 | 79.93 | 5.72 |
| 15 | 89.75 | 5.75 | 67.61 | 0.47 | 76.92 | 0.82 | 83.91 | 3.05 |
| 30 | 96.42 | 2.77 | 77.70 | 0.29 | 85.93 | 0.10 | 90.83 | 1.68 |
| 45 | 97.48 | 2.43 | 83.73 | 0.06 | 90.86 | 0.28 | 94.35 | 1.05 |
| 60 | 98.02 | 2.23 | 87.53 | 0.07 | 93.13 | 0.36 | 95.40 | 0.57 |

### Example 1-3: Formulation Using HPMC as Water-Soluble Polymer, Surfactant and Saccharide - Evaluation of Dissolution Pattern according to the Kind of Surfactant

Celecoxib and the excipients shown in Table 9 below were wet-kneaded, dried, granulated, mixed, and compressed into a tablet by wet-granulation method, and the tablet was subjected to an *in vitro* dissolution test.

**Table 9**

| Process | Function | Component name | YYC-301-1-31 | YYC-301-1-32 | YYC-301-1-33 | YYC-301-1-34 |
|---|---|---|---|---|---|---|
| | | | mg | mg | mg | mg |
| Kneading | Active ingredient | Celecoxib | 200 | 200 | 200 | 200 |
| | Excipient | mannitol100SD | 119.8 | 119.8 | 119.8 | 119.8 |
| | Excipient | Pharmacoat 603 | 5 | 5 | 5 | 5 |
| | Excipient | Sugar ester P-1570 | 5 | 5 | | |
| | Excipient | Labrafil M2125CS | | | 5 | |
| | Excipient | Castor oil | | | | 5 |
| | Disintegrant | Primellose | 6.8 | 6.8 | 6.8 | 6.8 |
| | Disintegrant | Mg-St | 3.4 | 3.4 | 3.4 | 3.4 |
| Solvent | | | Water | 50%EtOH | Water | Water |
| Sum | | | 340 | 340 | 340 | 340 |

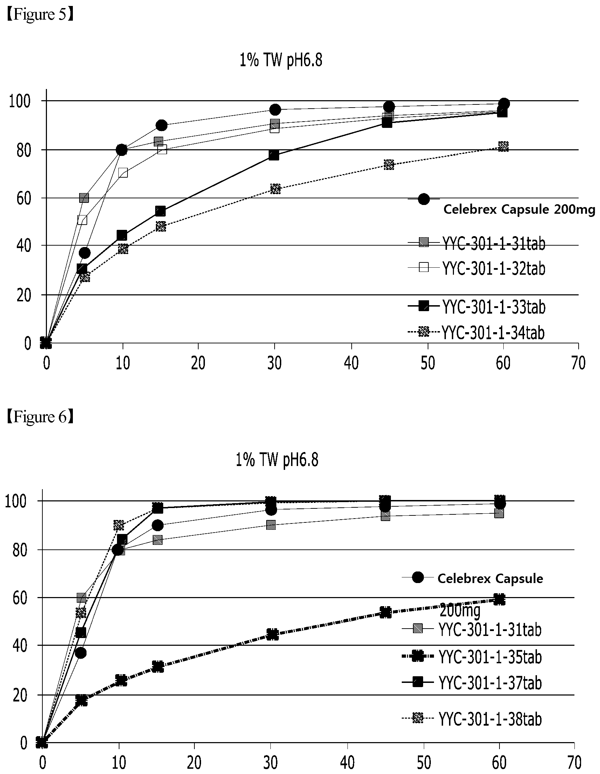

The celecoxib granules in Table 9 above were subjected to post-mixing and compressed into a tablet, and dissolution of the tablet was evaluated. The results are shown in Table 10 below and FIG. 5.

**Table 10**

| Time (min) | Celebrex capsule 200 mg | | YYC-301-1-31tab | | YYC-301-1-32tab | | YYC-301-1-33tab | | YYC-301-1-34tab | |
|---|---|---|---|---|---|---|---|---|---|---|
| | Dissolution rate | SD | Dissolution rate | SD | Dissolution rate | SD | Dissolution rate | SD | Dissolution rate | SD |
| 0 | 0.00 | 0.00 | 0.00 | 0.00 | 0.00 | 0.00 | 0.00 | 0.00 | 0.00 | 0.00 |
| 5 | 37.13 | 8.01 | 60.23 | 6.41 | 51.06 | 4.50 | 30.75 | 0.78 | 27.32 | 0.56 |
| 10 | 79.99 | 6.64 | 79.93 | 5.72 | 70.34 | 1.71 | 44.71 | 1.50 | 39.40 | 0.68 |
| 15 | 89.75 | 5.75 | 83.91 | 3.05 | 79.05 | 1.05 | 54.75 | 0.87 | 48.10 | 0.74 |
| 30 | 96.42 | 2.77 | 90.83 | 1.68 | 88.90 | 0.58 | 77.62 | 0.64 | 64.07 | 0.78 |
| 45 | 97.48 | 2.43 | 94.35 | 1.05 | 93.33 | 0.54 | 91.73 | 0.52 | 74.14 | 0.66 |
| 60 | 98.02 | 2.23 | 95.40 | 0.57 | 94.96 | 0.55 | 96.40 | 0.88 | 80.67 | 0.69 |

### Example 1-4: Formulation Using Water-Soluble Polymer, Sugar-ester P-1570 as Surfactant and Mannitol as Saccharide - Evaluation of Dissolution Pattern according to the Kind of Water-Soluble Polymer

Celecoxib and the excipients shown in Table 11 below were wet-kneaded, dried, granulated, mixed, and compressed into a tablet by wet-granulation method, and the tablet was subjected to an *in vitro* dissolution test.

**Table 11**

| Process | Function | ComponentName | YYC-301-1-31 | YYC-301-1-35 | YYC-301-1-37 | YYC-301-1-38 |
|---|---|---|---|---|---|---|
| | | | mg | mg | mg | mg |
| Kneading | Active ingredient | Celecoxib | 200 | 200 | 200 | 200 |
| | Excipient | mannitol100SD | 119.8 | 119.8 | 119.8 | 119.8 |
| | Excipient | Pharmacoat 603 | 5 | | | |
| | Excipient | Soluplus | | 5 | | |
| | Excipient | Kollidon VA64 | | | 5 | |
| | Excipient | HPC-L | | | | 5 |
| | Excipient | Sugar ester P-1570 | 5 | 5 | 5 | 5 |
| | Disintegrant | Primellose | 6.8 | 6.8 | 6.8 | 6.8 |
| | Disintegrant | Mg-St | 3.4 | 3.4 | 3.4 | 3.4 |
| Sum | | | 340 | 340 | 340 | 340 |

The celecoxib granules in Table 11 above were subjected to post-mixing and compressed into a tablet, and dissolution of the tablet was evaluated. The results are shown in Table 12 below and FIG. 6.

**Table 12**

| Time (min) | Celebrex capsule 200 mg | | YYC-301-1-31tab | | YYC-301-1-35tab | | YYC-301-1-37tab | | YYC-301-1-38tab | |
|---|---|---|---|---|---|---|---|---|---|---|
| | Dissolution rate | SD | Dissolution rate | SD | Dissolution rate | SD | Dissolution rate | SD | Dissolution rate | SD |
| 0 | 0.00 | 0.00 | 0.00 | 0.00 | 0.00 | 0.00 | 0.00 | 0.00 | 0.00 | 0.00 |
| 5 | 37.13 | 8.01 | 60.23 | 6.41 | 17.68 | 0.22 | 45.59 | 1.60 | 53.27 | 2.93 |
| 10 | 79.99 | 6.64 | 79.93 | 5.72 | 26.12 | 0.29 | 83.03 | 4.75 | 89.68 | 2.22 |
| 15 | 89.75 | 5.75 | 83.91 | 3.05 | 32.29 | 0.32 | 96.91 | 2.57 | 96.05 | 1.26 |
| 30 | 96.42 | 2.77 | 90.83 | 1.68 | 44.85 | 0.58 | 100.37 | 0.56 | 99.49 | 0.81 |
| 45 | 97.48 | 2.43 | 94.35 | 1.05 | 52.86 | 0.54 | 101.10 | 0.71 | 100.62 | 1.12 |
| 60 | 98.02 | 2.23 | 95.40 | 0.57 | 58.88 | 0.64 | 101.22 | 0.42 | 100.61 | 0.97 |

### Example 1-5: Formulation Using HPMC as Water-Soluble Polymer, Sugar Ester as Surfactant and Saccharide - Evaluation of Dissolution according to the Kind of Saccharide

Celecoxib and the excipients shown in Table 13 below were wet-kneaded, dried, granulated, mixed, and compressed into a tablet by wet-granulation method, and the tablet was subjected to an *in vitro* dissolution test.

**Table 13**

| Process | Function | Component Name | YYC-301-1-39 | YYC-301-1-36 | YYC-301-1-41 | YYC-301-1-44 |
|---|---|---|---|---|---|---|
| | | | mg | mg | mg | mg |
| Kneading | Active ingredient | Celecoxib | 200 | 200 | 200 | 200 |
| | Excipient | Pharmacoat603 | 5 | 5 | 5 | 5 |
| | Excipient | mannitol100SD | 119.8 | | | |
| | Excipient | HP-β-CD | | 119.8 | | |
| | Excipient | Glucose | | | 119.8 | |
| | Excipient | Emdex | | | | 119.8 |
| | Excipient | Sugar ester P-1570 | 5 | 5 | 5 | 5 |
| | Disintegrant | Primellose | 6.8 | 6.8 | 6.8 | 6.8 |
| | Lubricant | Mg-St | 3.4 | 3.4 | 3.4 | 3.4 |
| Sum | | | 340 | 340 | 340 | 340 |

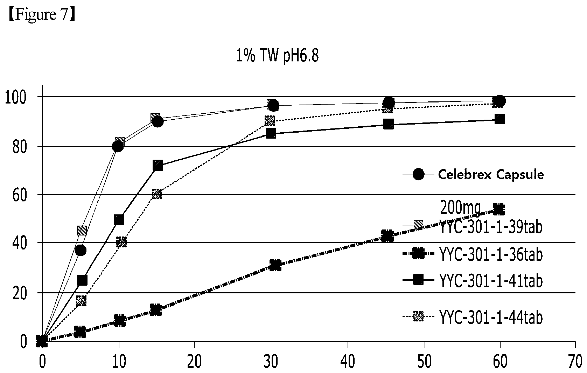

The celecoxib granules in Table 13bove were subjected to post-mixing and compressed into a tablet, and dissolution of the tablet was evaluated. The results are shown in Table 14 Table below and FIG. 7.

**Table 14**

| Time (min) | Celebrex capsule 200 mg | | YYC-301-1-39tab | | YYC-301-1-36tab | | YYC-301-1-41tab | | YYC-301-1-44tab | |
|---|---|---|---|---|---|---|---|---|---|---|
| | Dissolution rate | SD | Dissolution rate | SD | Dissolution rate | SD | Dissolution rate | SD | Dissolution rate | SD |
| 0 | 0.00 | 0.00 | 0.00 | 0.00 | 0.00 | 0.00 | 0.00 | 0.00 | 0.00 | 0.00 |
| 5 | 37.13 | 8.01 | 43.90 | 2.72 | 3.74 | 1.10 | 24.53 | 2.25 | 16.51 | 2.69 |
| 10 | 79.99 | 6.64 | 81.61 | 1.81 | 8.67 | 2.38 | 49.37 | 4.39 | 40.23 | 5.10 |
| 15 | 89.75 | 5.75 | 90.64 | 0.45 | 12.52 | 1.74 | 71.77 | 3.87 | 60.54 | 7.27 |
| 30 | 96.42 | 2.77 | 95.71 | 0.83 | 31.03 | 10.97 | 85.16 | 1.23 | 89.81 | 1.78 |
| 45 | 97.48 | 2.43 | 97.17 | 0.47 | 43.33 | 11.70 | 88.78 | 0.86 | 95.47 | 0.62 |
| 60 | 98.02 | 2.23 | 97.30 | 0.60 | 53.95 | 11.54 | 90.37 | 0.63 | 97.66 | 0.37 |

### Example 2: Sustained-Release Formulation of Water-Soluble Drug

In the following Tables, "YYC-301-1-(59+XX)" refers to a bilayered tablet comprising celecoxib compartment YYC-301-1-59 in Table 25 and tramadol compartment YYC-301-1-XX.

### Example 2-1: Formulation Using Ethylcellulose and Wax-Like Lipid Excipient - Evaluation of Dissolution Pattern according to the Amount of Wax-Like Lipid

Tramadol hydrochloride and the excipients shown in Table 15 below were wet-kneaded, dried, granulated, mixed, and compressed into a tablet by wet-granulation method, and the tablet was subjected to an *in vitro* dissolution test.

**Table 15**

| Function | Componentname | YYC-301-1-67 | YYC-301-1-68 | YYC-301-1-70 |
|---|---|---|---|---|
| | | 360mg/T | 400mg/T | 430mg/T |
| Active ingredient | Tramadol hydrochloride | 100.0 | 100.0 | 100.0 |
| Excipient | AqualonECN14 | 156.6 | 156.6 | 156.6 |
| Excipient | Compritol 888ATO | 80.0 | 117.4 | 145.45 |
| Binder | Aqualon EC N7 | 19.8 | 22.0 | 23.65 |
| Lubricant | Mg-St | 3.6 | 4.0 | 4.3 |
| Solvent | EtOH | 65.0mgT | 71.28mgT | 76.65mg/T |
| Process | | wet | wet | wet |
| Sum | | 360.0 | 400.0 | 430.0 |

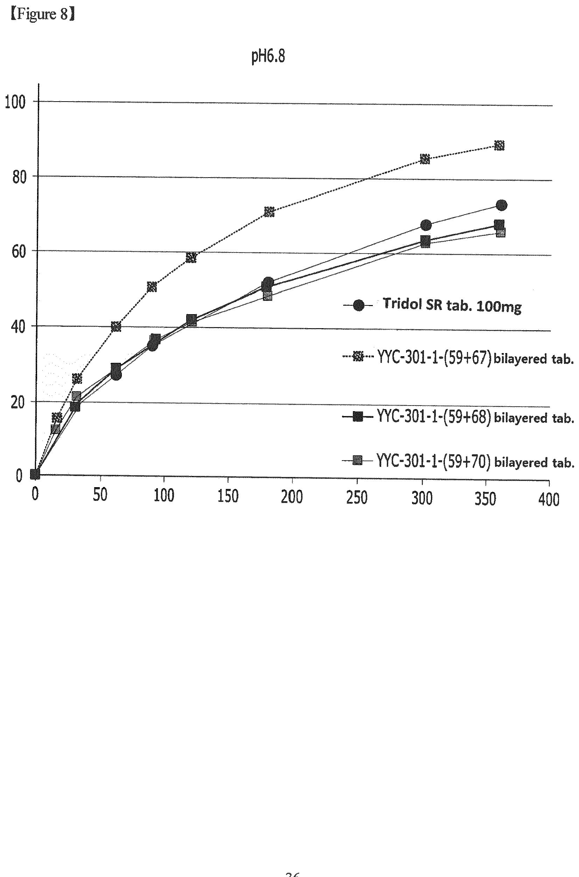

The results of evaluation of dissolution of the tablets shown in Table 15 above are shown in Table 16 below and FIG. 8.

**Table 16**

| Time (min) | Tridol sustained-release tablet 100 mg | | YYC-301-1-(59+67) bilayered tablet | | YYC-301-1-(59+68) bilayered tablet | | YYC-301-1-(59+70) bilayered tablet | |
|---|---|---|---|---|---|---|---|---|
| | Dissolution rate | SD | Dissolution rate | SD | Dissolution rate | SD | Dissolution rate | SD |
| 0 | 0.00 | 0.00 | 0.00 | 0.00 | 0.00 | 0.00 | 0.00 | 0.00 |
| 15 | 11.24 | 0.59 | 15.01 | 0.82 | 11.22 | 0.46 | 12.28 | 1.31 |
| 30 | 17.99 | 0.73 | 25.50 | 0.90 | 18.10 | 0.26 | 20.98 | 2.91 |
| 60 | 27.57 | 1.03 | 40.15 | 2.93 | 28.45 | 0.20 | 29.13 | 0.49 |
| 90 | 35.18 | 1.42 | 50.46 | 4.06 | 35.87 | 0.45 | 36.94 | 1.60 |
| 120 | 41.43 | 1.38 | 58.56 | 4.39 | 41.67 | 0.79 | 40.92 | 0.53 |
| 180 | 51.93 | 1.46 | 70.89 | 4.92 | 50.84 | 1.22 | 49.09 | 0.28 |
| 300 | 67.40 | 1.40 | 85.26 | 5.32 | 63.13 | 1.92 | 63.23 | 3.24 |
| 360 | 73.10 | 1.36 | 89.16 | 5.66 | 67.71 | 2.28 | 65.98 | 0.18 |

### Example 2-2: Formulation Using Ethylcellulose, Wax-Like Lipid Excipient - Evaluation of Dissolution Pattern according to the Amount of Ethylcellulose

Tramadol hydrochloride and the excipients shown in Table 17 below were wet-kneaded, dried, granulated, mixed, and compressed into a tablet by wet-granulation method, and the tablet was subjected to an *in vitro* dissolution test.

**Table 17**

| Function | Component name | YYC-301-1-67 | YYC-301-1-69 | YYC-301-1-71 |
|---|---|---|---|---|
| | | 360mg/T | 400mg/T | 430mg/T |
| Active ingredient | Tramadol hydrochloride | 100.0 | 100.0 | 100.0 |
| Excipient | AqualonECN14 | 156.6 | 194.0 | 222.05 |
| Excipient | Compritol 888ATO | 80.0 | 80.0 | 80.0 |
| Binder | Aqualon EC N7 | 19.8 | 22.0 | 23.65 |
| Lubricant | Mg-St | 3.6 | 4.0 | 4.0 |
| Solvent | EtOH | 65.0mgT | 71.28mgT | 76.65mg/T |
| Process | | wet | wet | wet |
| Sum | | 360.0 | 400.0 | 430.0 |

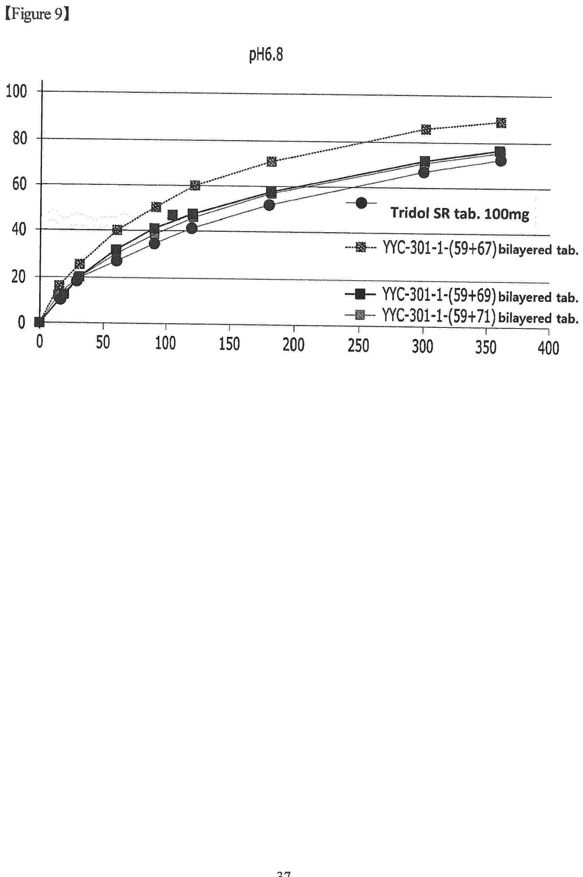

The results of evaluation of dissolution of the tablets shown in Table 17 above are shown in Table 18 below and FIG. 9.

**Table 18**

| Time (min) | Tridol sustained-release tablet 100 mg | | YYC-301-1-(59+67) bilayered tablet | | YYC-301-1-(59+69) bilayered tablet | | YYC-301-1-(59+71) bilayered tablet | |
|---|---|---|---|---|---|---|---|---|
| | Dissolution rate | SD | Dissolution rate | SD | Dissolution rate | SD | Dissolution rate | SD |
| 0 | 0.00 | 0.00 | 0.00 | 0.00 | 0.00 | 0.00 | 0.00 | 0.00 |
| 15 | 11.24 | 0.59 | 15.01 | 0.82 | 11.69 | 0.66 | 11.72 | 0.91 |
| 30 | 17.99 | 0.73 | 25.50 | 0.90 | 19.17 | 0.23 | 18.94 | 1.37 |
| 60 | 27.57 | 1.03 | 40.15 | 2.93 | 32.01 | 0.84 | 31.05 | 3.10 |
| 90 | 35.18 | 1.42 | 50.46 | 4.06 | 40.75 | 1.25 | 39.61 | 4.10 |
| 120 | 41.43 | 1.38 | 58.56 | 4.39 | 47.41 | 1.57 | 46.44 | 4.73 |
| 180 | 51.93 | 1.46 | 70.89 | 4.92 | 57.61 | 1.91 | 56.92 | 5.19 |
| 300 | 67.40 | 1.40 | 85.26 | 5.32 | 71.02 | 1.83 | 70.38 | 5.34 |
| 360 | 73.10 | 1.36 | 89.16 | 5.66 | 75.79 | 2.14 | - | - |

### Example 2-3: Formulation Using Ethylcellulose, Wax-Like Lipid Excipient - Evaluation of Dissolution Pattern according to the Kind of Wax-Like Lipid

Tramadol hydrochloride and the excipients shown in Table 19 below were wet-kneaded, dried, granulated, mixed, and compressed into a tablet by wet-granulation method, and the tablet was subjected to an *in vitro* dissolution test.

**Table 19**

| Function | Component name | YYC-301-1-68 | YYC-301-1-76 |
|---|---|---|---|
| | | 400mg/T | 400mg/T |
| Active ingredient | Tramadol hydrochloride | 100.0 | 100.0 |
| Excipient | AqualonECN14 | 156.6 | 156.6 |
| Excipient | Compritol 888ATO (glycerol dibehenate) | 117.4 | - |
| Excipient | Precirol ATO5 (glycerol distearate) | - | 117.4 |
| Binder | Aqualon EC N7 | 22.0 | 22.0 |
| Excipient | SMCC50 | - | - |
| Lubricant | Mg-St | 4.0 | 4.0 |
| Solvent | EtOH | 71.28mgT | 90.0mg/T |
| Process | | wet | wet |
| Sum | | 400.0 | 400.0 |

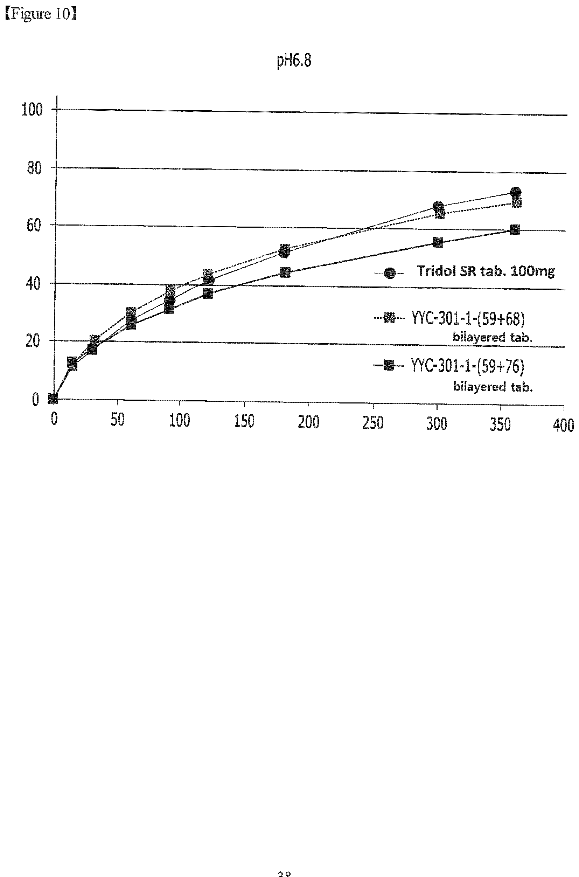

The results of evaluation of dissolution of the tablets shown in Table 19 above are shown in Table 20 below and FIG. 10.

**Table 20**

| Time (min) | Tridol sustained-release tablet 100 mg | | YYC-301-1-(59+68) bilayered tablet | | YYC-301-1-(59+76) bilayered tablet | |
|---|---|---|---|---|---|---|
| | Dissolution rate | SD | Dissolution rate | SD | Dissolution rate | SD |
| 0 | 0.00 | 0.00 | 0.00 | 0.00 | 0.00 | 0.00 |
| 15 | 11.24 | 0.59 | 10.71 | 2.22 | 12.36 | 0.58 |
| 30 | 17.99 | 0.73 | 19.67 | 1.17 | 17.67 | 0.65 |
| 60 | 27.57 | 1.03 | 29.81 | 1.13 | 25.54 | 0.65 |
| 90 | 35.18 | 1.42 | 37.51 | 1.44 | 31.51 | 0.70 |
| 120 | 41.43 | 1.38 | 43.59 | 1.90 | 36.46 | 0.73 |
| 180 | 51.93 | 1.46 | 52.53 | 1.89 | 44.27 | 0.77 |
| 300 | 67.40 | 1.40 | 65.20 | 2.43 | 55.47 | 0.91 |
| 360 | 73.10 | 1.36 | 69.90 | 2.61 | 59.88 | 1.06 |

### Example 2-4: Formulation Using Wax-Like Lipid Excipient - Evaluation of Dissolution Pattern according to the Proportions of MCC and Lubritab (Hydrogenated Castor Oil)

Tramadol hydrochloride and the excipients shown in Table 21 below were wet-kneaded, dried, granulated, mixed, and compressed into a tablet by wet-granulation method, and the tablet was subjected to an *in vitro* dissolution test.

**Table 21**

| Function | Component name | YYC-301-1-86 | YYC-301-1-87 | YYC-301-1-90 | YYC-301-1-91 |
|---|---|---|---|---|---|
| | | 400mg/T | 400mg/T | 400mg/T | 400mg/T |
| Active ingredient | Tramadol hydrochloride | 100.0 | 100.0 | 100.0 | 100.0 |
| Excipient | Lubritab | 82.4 | 102.4 | 62.4 | 42.4 |
| Excipient | Precirol ATO5 | 120.0 | 120.0 | 120.0 | 120.0 |
| Binder | Aqualon EC N7 | 22.0 | 22.0 | 22.0 | 22.0 |
| Excipient | SMCC90 | 61.6 | 41.6 | 81.6 | 101.6 |
| Excipient | Syloid | 10.0 | 10.0 | 10.0 | 10.0 |
| Lubricant | Mg-St | 4.0 | 4.0 | 4.0 | 4.0 |
| Solvent | EtOH | 90.0mg/T | 90.0mg/T | 75.0mg/T | 75.0mg/T |
| Process | | wet | wet | wet | wet |
| Sum | | 400.0 | 400.0 | 400.0 | 400.0 |

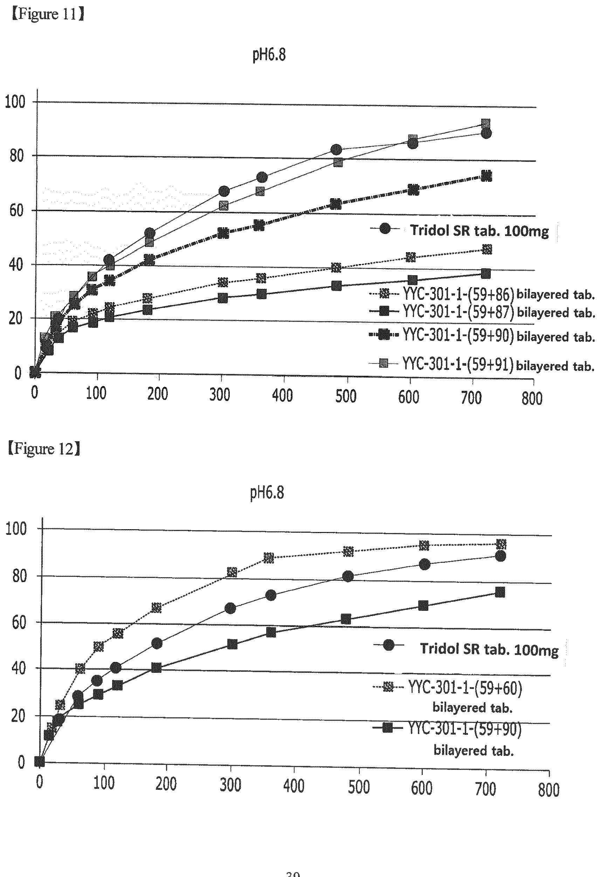

The results of evaluation of dissolution of the tablets shown in Table 21 above are shown in Table 22 below and FIG. 11.

**Table 22**

| Time (min) | Tridol sustained-release tablet 100 mg | | YYC-301-1-(59+86) bilayered tablet | | | |
|---|---|---|---|---|---|---|
| | Dissolution rate | SD | Dissolution rate | SD | | |
| 0 | 0.00 | 0.00 | 0.00 | 0.00 | | |
| 15 | 11.24 | 0.59 | 9.19 | 0.79 | | |
| 30 | 17.99 | 0.73 | 13.28 | 1.30 | | |
| 60 | 27.57 | 1.03 | 18.81 | 0.18 | | |
| 90 | 35.18 | 1.42 | 21.61 | 0.32 | | |
| 120 | 41.43 | 1.38 | 24.13 | 0.24 | | |
| 180 | 51.93 | 1.46 | 27.96 | 0.24 | | |
| 300 | 67.40 | 1.40 | 33.91 | 0.25 | | |
| 360 | 73.10 | 1.36 | 36.09 | 0.24 | | |
| 480 | 81.85 | 0.85 | 40.27 | 0.39 | | |
| 600 | 87.21 | 0.79 | 43.99 | 0.38 | | |
| 720 | 90.99 | 0.23 | 47.36 | 0.49 | | |

| Time (min) | YYC-301-1-(59+87) bilayered tablet | | YYC-301-1-(59+90) bilayered tablet | | YYC-301-1-(59+91) bilayered tablet | |
|---|---|---|---|---|---|---|
| | Dissolution rate | SD | Dissolution rate | SD | Dissolution rate | SD |
| 0 | 0.00 | 0.00 | 0.00 | 0.00 | 0.00 | 0.00 |
| 15 | 8.81 | 0.33 | 11.37 | 0.30 | 12.61 | 0.49 |
| 30 | 12.57 | 0.81 | 17.37 | 1.55 | 20.24 | 0.10 |
| 60 | 16.42 | 0.63 | 25.04 | 1.16 | 29.12 | 0.47 |
| 90 | 18.78 | 0.53 | 30.49 | 0.77 | 35.48 | 0.57 |
| 120 | 20.76 | 0.46 | 34.76 | 0.45 | 40.71 | 0.60 |
| 180 | 23.80 | 0.43 | 41.53 | 0.20 | 49.25 | 0.76 |
| 300 | 28.07 | 0.37 | 51.88 | 0.48 | 62.49 | 1.25 |
| 360 | 29.80 | 0.54 | 55.89 | 0.42 | 68.25 | 1.47 |
| 480 | 32.92 | 0.43 | 63.03 | 0.65 | 79.04 | 1.77 |
| 600 | 35.75 | 0.67 | 69.25 | 0.73 | 87.95 | 1.42 |
| 720 | 38.42 | 0.55 | 75.02 | 0.86 | 93.79 | 1.29 |

### Example 3: Example of Celecoxib Dissolution Interference of Water-Soluble Matrix

Each of tramadol hydrochloride and celecoxib and the excipients shown in the following Tables were wet-kneaded, dried, granulated, mixed, and compressed into a tablet by wet-granulation method, and the tablet was subjected to an *in vitro* dissolution test.

**Table 23: Tramadol formulation**

| Function | Component name | YYC-301-1-60 | YYC-301-1-90 |
|---|---|---|---|
| | | 400mg/T | 400mg/T |
| Active ingredient | Tramadol hydrochloride | 100.0 | 100.0 |
| Excipient | PH101 | 100.0 | |
| Excipient | SMCC90 | | 81.6 |
| Excipient | Starch1500 | 37.3 | |
| Binder | EC(100cP) | 6.0 | |
| Binder | EC(7cP) | | 22.0 |
| Excipient | HPMC2910 15000SR | 20.0 | |
| Excipient | Precirol ATO5 | | 120.0 |
| Excipient | Lubritab | | 62.4 |
| Excipient | Syloid | | 10.0 |
| Lubricant | Mg-St | 2.7 | 4.0 |
| Process | | wet | wet |
| Sum | | 266.0 | 400.0 |

**Table 25: Celecoxib formulation according to one embodiment of the present invention**

| Function | Component name | YYC-301-1-59 |
|---|---|---|
| | | 360mg/T |
| Active ingredient | Celecoxib | 200.0 |
| Disintegrant | Primellose | 16.2 |
| Excipient | D-mannitol | 125.2 |
| Excipient | Sugar ester P-1570 | 5.0 |
| Binder | HPC-L | 5.0 |
| Disintegrant | Kollidon CL | 5.0 |
| Lubricant | Mg-St | 3.6 |
| Sum | | 360 |

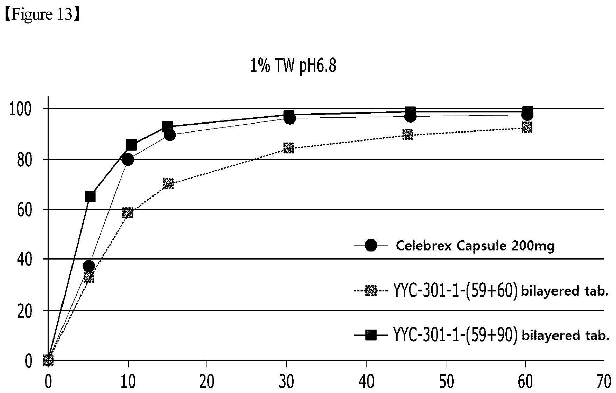

The results of dissolution evaluation are shown in Tables 26 and 27 below and FIGS. 12 and 13.

**Table 26**

| Time (min) | Tridol sustained-release tablet 100 mg | | YYC-301-1-(59+60) bilayered tablet | | YYC-301-1-(59+90) bilayered tablet | |
|---|---|---|---|---|---|---|
| | Dissolution rate | SD | Dissolution rate | SD | Dissolution rate | SD |
| 0 | 0.00 | 0.00 | 0.00 | 0.00 | 0.00 | 0.00 |
| 15 | 11.24 | 0.59 | 15.00 | 0.52 | 11.37 | 0.30 |
| 30 | 17.99 | 0.73 | 24.97 | 1.48 | 17.37 | 1.55 |
| 60 | 27.57 | 1.03 | 39.46 | 4.54 | 25.04 | 1.16 |
| 90 | 35.18 | 1.42 | 49.32 | 5.51 | 30.49 | 0.77 |
| 120 | 41.43 | 1.38 | 55.76 | 4.66 | 34.76 | 0.45 |
| 180 | 51.93 | 1.46 | 66.66 | 3.47 | 41.53 | 0.20 |
| 300 | 67.40 | 1.40 | 81.89 | 0.77 | 51.88 | 0.48 |
| 360 | 73.10 | 1.36 | 86.32 | 0.21 | 55.89 | 0.42 |
| 480 | 81.85 | 0.85 | 91.82 | 0.08 | 63.03 | 0.65 |
| 600 | 87.21 | 0.79 | 94.77 | 0.70 | 69.25 | 0.73 |
| 720 | 90.99 | 0.23 | 96.36 | 0.79 | 75.02 | 0.86 |

**Table 27**

| Time (min) | Celebrex capsule 200 mg | | YYC-301-1-(59+60) bilayered tablet | | YYC-301-1-(59+90) bilayered tablet | |
|---|---|---|---|---|---|---|
| | Dissolution rate | SD | Dissolution rate | SD | Dissolution rate | SD |
| 0 | 0.00 | 0.00 | 0.00 | 0.00 | 0.00 | 0.00 |
| 5 | 37.13 | 8.01 | 31.97 | 10.84 | 64.43 | 5.34 |
| 10 | 79.99 | 6.64 | 57.37 | 9.25 | 85.89 | 6.34 |
| 15 | 89.75 | 5.75 | 69.77 | 4.84 | 93.19 | 1.62 |
| 30 | 96.42 | 2.77 | 83.55 | 2.99 | 97.43 | 0.65 |
| 45 | 97.48 | 2.43 | 89.38 | 2.77 | 98.71 | 0.17 |
| 60 | 98.02 | 2.23 | 92.59 | 2.63 | 97.90 | 0.70 |

## Claims

1. An oral solid pharmaceutical composition comprising: a first compartment of immediate-release comprising celecoxib; and a second compartment of sustained-release comprising tramadol,
wherein the first compartment comprises a water-soluble polymer, a surfactant and a saccharide,
wherein the second compartment comprises a water-insoluble polymer and a wax-like lipid,
wherein the water-soluble polymer is one or more selected from among hydroxypropyl cellulose, hydroxypropyl methylcellulose and a vinylpyrrolidone-vinyl acetate copolymer,
wherein the saccharide comprises one or more selected from among mannitol, maltitol, lactitol, ribitol, inositol, xylitol, maltotritol and glucose,
wherein the water-insoluble polymer comprises one or more selected from among cellulose-based polymers, and
wherein the wax-like lipid comprises one or more selected from among glycerol stearate, glycerol behenate, glycerol palmitostearate, fatty acid macrogol glyceride, diethylene glycol monoethyl ether, glyceryl monocaprylate and hydrogenated castor oil.

2. The pharmaceutical composition of claim 1, wherein the surfactant comprises one or more selected from among polyoxyglyceride, polyoxyethylene sorbitan fatty acid ester, sodium lauryl sulfate, glyceryl fatty acid ester, fatty acid macrogol glyceride, diethylene glycol monoethyl ether, and sucrose fatty acid ester.

3. The pharmaceutical composition of claim 1, wherein the water-soluble polymer and the surfactant is contained in an amount of 0.1 to 20 wt% of the total weight of the first compartment.

4. The pharmaceutical composition of claim 1, wherein the saccharide is contained in an amount of 10 to 50 wt% of the total weight of the first compartment.

5. The pharmaceutical composition of claim 1, wherein the water-insoluble polymer comprises one or more selected from ethyl cellulose and cellulose acetate.

6. The pharmaceutical composition of claim 1, wherein the wax-like lipid and the water-insoluble polymer is contained in an amount of 1 to 60 wt% based on the total weight of the second compartment.

## Patentansprüche

1. Orale, feste pharmazeutische Zusammensetzung, umfassend: ein erstes Kompartiment zur sofortigen Freisetzung umfassend Celecoxib und ein zweites Kompartiment zur verzögerten Freisetzung umfassend Tramadol,
wobei das erste Kompartiment ein wasserlösliches Polymer, einen oberflächenaktiven Stoff und ein Saccharid umfasst;
wobei das zweite Kompartiment ein wasserunlösliches Polymer und ein wachsartiges Lipid umfasst,
wobei das wasserlösliche Polymer ein oder mehrere ausgewählt aus Hydroxypropylcellulose, Hydroxypropylmethylcellulose und einem Vinylpyrrolidon-Vinylacetat-Copolymer ist,
wobei das Saccharid ein oder mehrere ausgewählt aus Mannitol, Maltitol, Lactitol, Ribitol, Inositol, Xylitol, Maltotritol und Glucose umfasst,
wobei das wasserunlösliche Polymer ein oder mehrere ausgewählt aus cellulosebasierten Polymeren umfasst,
wobei das wachsartige Lipid ein oder mehrere ausgewählt aus Glycerinstearat, Glycerinbehenat, Glycerinpalmitostearat, Fettsäure-macrogol-glycerid, Diethylenglycolmonoethylether, Glycerylmonocaprylat und hydriertem Rizinusöl umfasst.

2. Pharmazeutische Zusammensetzung nach Anspruch 1, wobei der oberflächenaktive Stoff ein oder mehrere ausgewählt aus Polyoxyglycerid, Polyoxyethylensorbitanfettsäureester, Natriumlaurylsulfat, Glycerylfettsäureester, Fettsäure-macrogol-glycerid, Diethylenglycolmonoethylether und Saccharosefettsäureester umfasst.

3. Pharmazeutische Zusammensetzung nach Anspruch 1, wobei das wasserlösliche Polymer und der oberflächenaktive Stoff in einem Anteil von 0,1 bis 20 Gew.-% des Gesamtgewichts des ersten Kompartiments enthalten sind.

4. Pharmazeutische Zusammensetzung nach Anspruch 1, wobei das Saccharid in einem Anteil von 10 bis 50 Gew.-% des Gesamtgewichts des ersten Kompartiments enthalten ist.

5. Pharmazeutische Zusammensetzung nach Anspruch 1, wobei das wasserunlösliche Polymer ein oder mehrere ausgewählt aus Ethylcellulose und Celluloseacetat umfasst.

6. Pharmazeutische Zusammensetzung nach Anspruch 1, wobei das wachsartige Lipid und das wasserunlösliche Polymer in einem Anteil von 1 bis 60 Gew.-% des Gesamtgewichts des zweiten Kompartiments enthalten sind.

## Revendications

1. Une composition pharmaceutique solide à usage oral comprenant : un premier compartiment de libération immédiate comprenant du célécoxib ; et un deuxième compartiment de libération prolongée comprenant du tramadol,
dans laquelle le premier compartiment comprend un polymère soluble dans l'eau, un tensioactif et un saccharide,
dans laquelle le deuxième compartiment comprend un polymère insoluble dans l'eau et un lipide de type cire,
dans laquelle le polymère soluble dans l'eau est un ou plusieurs choisis parmi l'hydroxypropylcellulose, l'hydroxypropylméthylcellulose et un copolymère de vinylpyrrolidone-acétate de vinyle,
dans laquelle le saccharide comprend un ou plusieurs parmi le mannitol, le maltitol, le lactitol, le ribitol, l'inositol, le xylitol, le maltotritol et le glucose,
dans laquelle le polymère insoluble dans l'eau comprend un ou plusieurs choisis parmi les polymères à base de cellulose, et
dans laquelle le lipide de type cire comprend un ou plusieurs choisis parmi le stéarate de glycérol, le béhénate de glycérol, le palmitostéarate de glycérol, un glycéride de macrogol et d'acide gras, l'éther monoéthylique de diéthylèneglycol, le monocaprylate de glycéryle et l'huile de ricin hydrogénée.

2. La composition pharmaceutique selon la revendication 1, dans laquelle le tensioactif comprend un ou plusieurs choisis parmi un polyoxyglycéride, un ester d'acide gras et de sorbitan polyoxyéthyléné, le laurylsulfate de sodium, un ester d'acide gras et de glycéryle, un glycéride de macrogol et d'acide gras, l'éther monoéthylique de diéthylèneglycol, et un ester d'acide gras de saccharose.

3. La composition pharmaceutique selon la revendication 1, dans laquelle le polymère soluble dans l'eau et le tensioactif sont contenus en une quantité de 0,1 à 20 % en poids du poids total du premier compartiment.

4. La composition pharmaceutique selon la revendication 1, dans laquelle le saccharide est contenu en une quantité de 10 à 50 % en poids du poids total du premier compartiment.

5. La composition pharmaceutique selon la revendication 1, dans laquelle le polymère insoluble dans l'eau comprend un ou plusieurs choisis parmi l'éthylcellulose et l'acétate de cellulose.

6. La composition pharmaceutique selon la revendication 1, dans laquelle le lipide de type cire et le polymère insoluble dans l'eau sont contenus en une quantité de 1 à 60 % en poids par rapport au poids total du deuxième compartiment.
